# EUROPEAN PATENT SPECIFICATION

(11) **EP 3 100 837 B1**
(45) Date of publication and mention of the grant of the patent: **26.07.2023**
(21) Application number: 16020173.7
(22) Date of filing: 06.05.2016
(51) Int. Cl.: B28B 23/00

(54) **CEMENTITIOUS MATERIAL STRUCTURE WITH SENSORS, MANUFACTURING METHOD AND OPERATION METHOD THEREOF**
ZEMENTARTIGE MATERIAL STRUKTUR MIT SENSOREN, DEREN HERSTELLUNGSVERFAHREN UND DEREN BETRIEBSVERFAHREN
STRUCTURE DE MATÉRIAU CIMENTAIRE AVEC CAPTEURS, SA MÉTHODE DE FABRICATION ET SA MÉTHODE D'OPÉRATION

(30) Priority: 07.05.2015 PT 2015108448
(43) Date of publication of application: 07.12.2016
(73) Proprietor: Secil-Companhia Geral de Cal e Cimento S.A., 1600-079 Lisboa (PT); Centi - Centro de Nanotecnologia e Materiais Tecnicos Funcionais e Inteligentes, 4760-034 Vila Nova de Famalicão (PT)
(72) Inventor: Carvalho Gomes, João Manuel, 4700-832 BRAGA (PT); Marques Pessoa, Ricardo Daniel, 4405-744 VILA NOVA DE GAIA (PT); Vasco, Gonçalves Pimenta Machado, 4750-879 BARCELOS (PT); Da Fonseca E Branquinho De Pais Monteiro, Joana, 3520-058 NELAS (PT); Poças Gonçalves, José Joaquim, 4710-432 BRAGA (PT); Silvestre Mendes Pinto De Moura, Bruna Gabriela, 4760-292 VILA NOVA DE FAMALICÃO (PT); Pinto Lopes, Jaime Rafael, 4460-080 GUIFÕES (PT); Da Fonseca, Joana Diniz, 4425-642 MAIA (PT); De Sequeira Serra Nunes, Ângela Maria Jesus, 2925-181 AZEITÃO (PT); Vermelhudo, Vitor, 2925-181 AZEITÃO (PT)
(74) Representative: Pereira da Cruz, Joao

(56) References cited:
- WO-A2-2006/138607
- KR-B1- 101 336 683
- US-A1- 2010 026 665
- US-A1- 2013 187 900
- US-B1- 8 532 815

## Description

### FIELD OF THE INVENTION

The present invention relates to the field of construction, involving knowledge of building materials and sensors technologies. More specifically, it involves cementitious materials and waterproofing materials, among others, as well as different technologies of contact or proximity sensors. The intersection of these technologic fields allows achieving constructive structures with different shapes, functions and dimensions, which interact with external elements.

Said cementitious materials are suitable for any kind of constructions, such as construction or rehabilitation, either inside or outside, of buildings, roads, street furniture, among others.

The object of the invention also relates to the development of sensors suitable for interacting with human users.

Thus, the present invention aims to introduce interactivity in cementitious material structures, through the integration of sensors during the concreting process. These structures have application in the construction/rehabilitation of buildings, in highways, street furniture, hospital facilities, among others.

### BACKGROUND OF THE INVENTION

The closest antecedents of the present invention are found in the internal parameter monitoring systems of constructive structures, for continuous monitoring purposes and, therefore, predictive maintenance.

Such systems are intended to monitor the response of structures over time, either in the construction phase itself or during its lifetime, by placing sensors on the surface of the cementitious structures, by drilling the structure and by placing the sensors on the inside, or by placing the sensors inside during its construction.

The latter type of systems is the one that matters for purposes of the present invention, in which sensors are embedded into the structure during its construction and become part of the structure itself.

Patent application publication No. WO 2007025172A2 discloses a system for monitoring the healing process of concrete, in which devices with sensing and wireless communication capabilities are placed inside the concrete during concreting, allowing to monitor the temperature at different points inside the structure, while concrete is drying.

Patent application publication No. CN 102255959A discloses a system in which devices with strain gauges and temperature sensors and having wireless communication capability are embedded in a concrete structure during concreting, so that they monitor parameters which allow to know the conservation state of the structure throughout its lifetime.

WO 2006/138607 A2 discloses a cementitious material structure with sensors according to the preamble of claim 1.

### TECHNICAL PROBLEMS SOLVED

In the present invention, it was intended to monitor external parameters and not internal structure parameters. With this change, it is intended to obtain interaction with the outside of the structure, in solutions of interactivity with a human user. Thus, the configuration, the arrangement, the sensor type and other features are distinct from those known in prior art, in a way that sensors embedded into the cementitious structures can detect variables external to the structure.

On the other hand, it is necessary to carry out a process different from those known in prior art to obtain an encapsulation, and therefore durability and reliability such that allow a proper operation of the sensors set over time, including providing resistance to the concreting process of the cementitious material structures.

Additionally, with the present invention it is intended to provide contactless user interfaces by making use of porosity characteristics of the cementitious materials

### SUMMARY OF THE INVENTION

It is therefore the object of the present invention a structure of cementitious material with sensors according to claim 1.

Such a configuration is related to the sensing characteristics of the cementitious material structure. It is intended to measure a certain physical variable along the surface of the cementitious material structure, or part of it, thus defining an area over which the variable is detected, resulting in an adequate sensitive surface for monitoring a certain variable over the outer surface (4) of the structure.

According to the invention, the cementitious material structure with sensors further includes a waterproofing material layer (3) covering the set of the plurality of sensing elements (1) and at least one substrate (2).

The waterproofing material layer (3) allows the sensing elements (1) and the substrate (2), which typically are barely suitable to be involved with cementitious material, to be involved by it, thus allowing an external finishing only in cementitious materials while maintaining the robustness and reliability characteristics of the set of sensors. The waterproofing material layer (3) is therefore placed between the set of the plurality of sensing elements (1) together with the at least one substrate (2) and the cementitious material.

In this configuration, the plurality of sensing elements (1) is printed on the substrate (2). To make possible this configuration, the sensing elements (1) are printed on the substrate (2) by means of different possible techniques.

Said sensitive surface is also involved in a waterproofing material, which encapsulates it in order to withstand the concreting process and throughout the lifetime of the structure, and involved in cementitious material, which provides a finishing that hides the plurality of sensing elements (1) and that corresponds to the outer surface (4) of the structure.

It is also an object of the present invention a manufacturing method of the cementitious material structure with sensors according to claim 10.

Through the method of the present invention, it is possible to obtain a cementitious material structure with sensors, which complies with said capability requirements for monitoring variables on the surface of the structure, and having a suitable encapsulation for the durability and reliability of the sensing elements (1).

This method further includes, for obtaining some of the configurations, the following step performed between steps a) and b):
- encapsulation of the set of the plurality of sensing elements (1) and the at least one substrate (2) with a waterproofing material (3).

### DESCRIPTION OF THE FIGURES

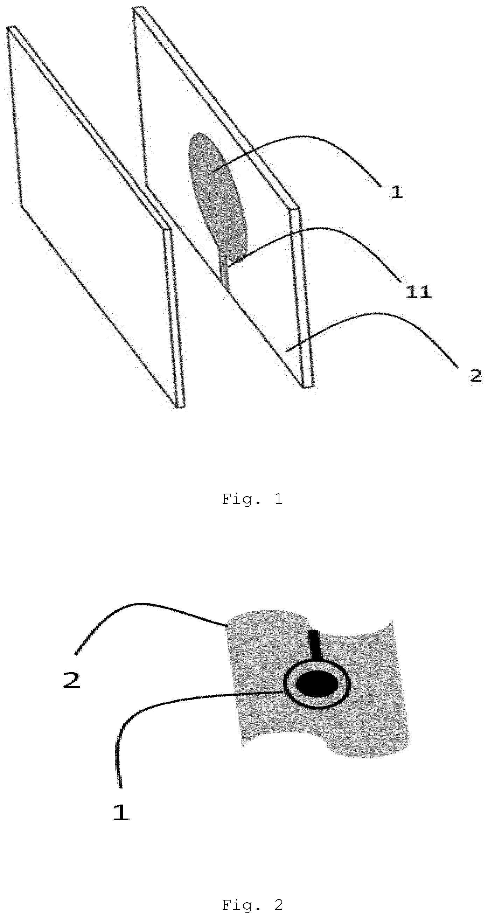

Figure 1 - representation of a cementitious material structure with sensors according to the present invention, showing a plurality of sensing elements (1), a substrate (2) on which they are integrated - printed in the case of the figure - and a base of cementitious material on which the substrate (2) is placed. It is also represented the cementitious material which covers and fills this set (slab shown at left) as well as one sensing element extension (11) for connection to the other electronic elements with functions such as signal conditioning or signal processing.
Figure 2 - another representation of a cementitious material structure with sensors according to the present invention, showing a plurality of sensing elements (1), a substrate (2) on which they are printed, and a base of cementitious material on which the substrate (2) is placed.

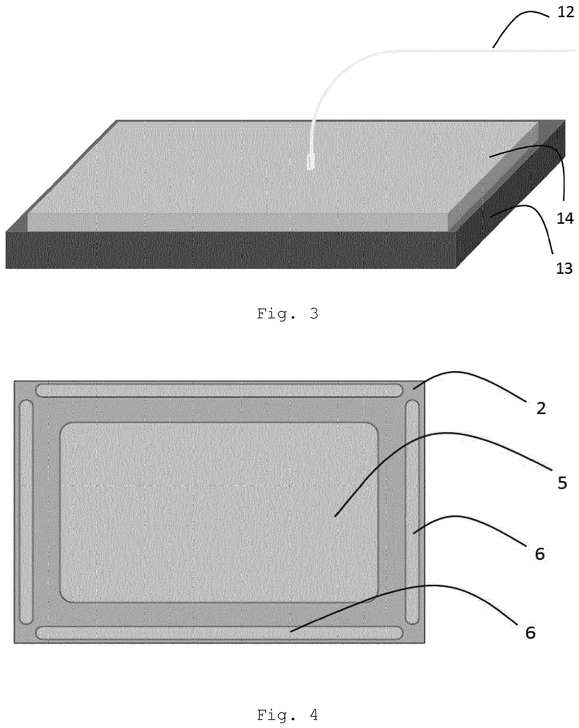

Figure 3 - representation of a set of substrate (2) with a plurality of sensing elements (1) - which define a base assembly (14) (these elements are not shown) - covered by cementitious material and placed in a mold for filling with cementitious materials (13). It is also represented a connection cable to power means (12).
Figure 4 - representation of the mesh of sensing elements (1) printed on a substrate (2), which allows to map the area where it is held the touch and/or where a user approaches to the structure of cementitious material with sensors. This action activates the closest sensing element (1), initiating an activation process of at least one actuator element (10) or remote communications element. Four peripheral sensing elements (6) and a central sensing element (5) are visible.

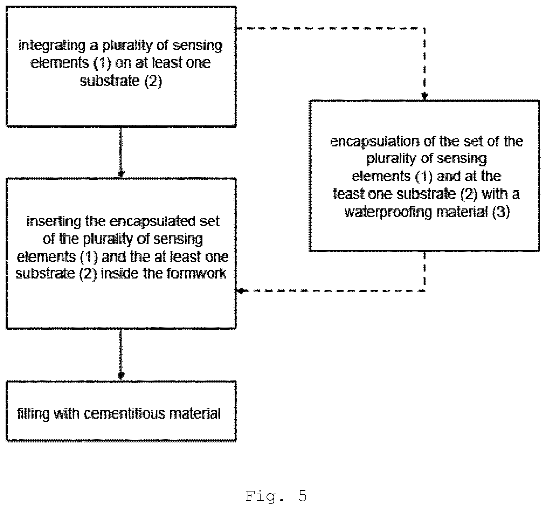

Figure 5 - representation of a manufacturing method not forming part of the present invention. The step of encapsulation with 5 waterproofing material (3) is shown as optional.

### DETAILED DESCRIPTION OF THE INVENTION

The present invention allows any cementitious material structure, for instance a concrete wall, becomes interactive with the outside, specifically incorporating a plurality of sensing elements (1) therein.

As mentioned before, systems are known that enable the incorporation of sensors into cementitious material structures, being however suitable for monitoring variables related to the structure condition.

Thus, the present invention comprises a configuration different from those of prior art and includes a cementitious material structure with sensors comprising a plurality of sensing elements (1) arranged on at least one substrate (2), and wherein the set of the plurality of sensing elements (1) with the at least one substrate (2) is involved in cementitious material.

The set of the plurality of sensing elements (1) with the at least one substrate (2) included in the structure is covered by a waterproofing material layer (3) which separates it from the involving cementitious material. This waterproofing material layer (3) allows said set to be encapsulated so that it resists the concreting process and remains in operation during the lifetime of the structure. The set of the plurality of sensing elements (1), with the at least one substrate (2) and with the involving cementitious materials is called base assembly (14) .

Additionally, this structure has a configuration in which the plurality of sensing elements (1) is arranged on a surface substantially parallel to an outer surface (4) of the cementitious material structure with sensors.

Such a configuration enables the alignment of the contact or outer interaction surface with the surface with sensors, thereby achieving the measurement of the variable along a particular area with sensors.

In one embodiment of the present invention, the plurality of sensing elements (1) is printed on the at least one substrate (2). This embodiment is highly suitable to create cementitious material structures with sensors *in situ* and does not need a previous preparation of the sensing elements (1). Through different methods that are subject of the present invention, it is quite simple and versatile to create structures with sensors based on the cementitious material, making any structure a potential point of interaction.

Also in this embodiment, and more specifically, the at least one substrate (2) is a substrate of a non-cementitious material, such as a polymeric material, paper, derivatives of paper, ceramic or glass. In case the substrate (2) is polymer, it may consist of polyethylene terephthalate (PET) or polyethylene naphthalate (PEN). The substrate (2) of a non-cementitious material thus consists of materials compatible with roller or sheets systems.

These substrates present characteristics of dimensional stability, levelled surface, temperature resistance, namely till 160°C, and moisture resistance.

Alternatively to a non-cementitious substrate (2), the at least one substrate (2) consists in the cementitious material itself or in a micro concrete layer, and the printing surface of the plurality of sensing elements (1) in the cementitious material forms a substantially finite plane parallel to the outer surface (4) of the structure. This type of substrate (2) is shaped in a mold for filling with cementitious material (13).

The sensors making part of the plurality of sensing elements (1) may consist in capacitive sensors, inductive sensors, sensors of electro-dermal activity, blood volume pulse, temperature, luminosity, sound, or a combination thereof, among others.

In the case of sensing elements (1) consisting of blood volume pulse (BVP) sensors of optoelectronic technology, the cementitious material structure with sensors additionally includes optical fibers arranged in such a way that one of the ends of each optical fiber is at the outer surface (4) of the structure and the other end is at the surface of each sensing element (1). This innovative configuration allows the sensing elements (1), which include a light source and a photodetector, to be encapsulated in the cementitious materials while simultaneously measuring BVP signals from the outside of the structure.

Preferably, the blood volume pulse (BVP) sensors of optoelectronic technology are not printed, but are rather prefabricated by one of the methods known in prior art.

In a most complex aspect of the present invention, it is intended to obtain a touchpad type interface destined to a human user.

Thus, the present invention includes a configuration of the cementitious material structure with sensors in which the plurality of sensing elements (1) is arranged in a mesh, allowing interaction with the user or the external element through the detection by different sensing elements (1) of the mesh. The mesh configures a plane parallel to the outer surface (4).

In a further possible embodiment of the mesh configuration, the cementitious material structure with sensors includes several planes parallel to each other and to the outer surface, having a plurality of sensing elements (1) and defining a three-dimensional mesh of sensing elements (1).

In an embodiment alternative or additional to the previous one, the plurality of sensing elements (1) of the cementitious material structure with sensors is arranged in such a way that a central sensing element (5) is surrounded by at least two peripheral sensing elements (6), arranged on a line which is part of a plane parallel to the outer surface (4) of the cementitious material structure with sensors.

In an embodiment additional to the previous one, the cementitious material structure with sensors includes at least four peripheral sensing elements (6) arranged in pairs in at least two perpendicular imaginary lines, which are part of a plane parallel to the outer surface (4) of the cementitious materials structure with sensors and intersect at midpoint of the central sensing element (5).

In both embodiments, the cementitious material structure with sensors allows the detection of the movements of the user over the surface with sensors, through individualized sensing elements (1).

In the case of tracking the approach of a detectable element by the sensing elements (1), it is possible to detect the exact position of the change in the sensing elements (1) and therefore detect the position of the element that caused the change.

For the treatment of data from the plurality of sensing elements (1), the present invention includes signal conditioning means (7), at least one microcontroller (8) and optionally local and/or remote communication means (9). The signal conditioning means (7) is connected to the plurality of sensing elements (1), being suitable to convert and adapt the signals from the sensing elements (1) into analogue/digital inputs of the at least one microcontroller (8), which processes the data and sends it via digital/analog communication to a wired/wireless central system.

In order for the structure that is object of the present invention to present the information, thus providing said interactivity, the cementitious material structure with sensors further includes, in one embodiment, at least one actuator element (10).

Therefore, the processing of the signals read by the integrated sensors allows generating different outputs in the at least one actuator element (10). This could consist of sound generating means, at least one light emitting diode (LED) or at least a lamp, an output controlling the activation of a device, or a screen displaying the signal read by the sensor, among others.

The analysis of data from the plurality of sensing elements (1) and the consequent actuation on the at least one actuator element (10) may be carried out *in situ* or remotely, as the local and/or remote communication means (9) is suitable for connecting to a global management control system, which allows a customized interaction with the user.

In the case of the at least one actuator element (10) consists of a LED or another type of light emitting means, and since in the present invention the electronics is integrated inside the concrete, the conduction of the light up to the outer surface (4) is made through a optical fiber which is also integrated into the cementitious material.

The same principle applies in the case in which the sensing elements (1) are, for example, blood volume pulse (BVP) sensors, wherein each fiber filament contacts, at one end, with the finger of the user, and with the light receiver or emitter located at the other end.

The power supply of the electronic components embedded in the cementitious material structure with sensors consists of finite supply equipment - primary or secondary batteries - or direct connection to the mains, including in this case AC/DC conversion means. The power supply of the electronic elements set which is part of the cementitious material structure of the present invention is performed using a power cable connecting to power means (12), in any of the alternatives listed.

In the case of sensing through printed sensing elements (1) on the at least one substrate (2), these sets are laminated and/or coated with different materials in order to obtain electrical, mechanical and chemical protection after printing and before integration in the concrete structure. As protective materials, waterproof materials and materials with good mechanical and chemical resistance are considered in order to resist the concreting process of the concrete parts. The encapsulation of the printed sensors with these materials can be made through lamination, and/or heat sealing, and/or slot die, and/or doctor blade, and/or knife-over-edge, and/or screen printing, and/or spray, of a polymeric material curable by ultraviolet (UV) light and/or temperature.

It is also an object of the present invention a manufacturing method of the cementitious material structure with sensors which includes the following steps:
a) integrating a plurality of sensing elements (1) on at least one substrate (2);
b) inserting the encapsulated set of the plurality of sensing elements (1) and the at least one substrate (2) inside the formwork;
c) filling with cementitious material.

According to the invention, the manufacturing method of the present invention further includes the following step, performed after step a):
- encapsulation of the set of the plurality of sensing elements (1) and the at least one substrate (2) with a waterproofing material (3).

Still more specifically, regarding the configuration of cementitious material structure with sensors in which the sensing elements (1) are printed on at least one substrate (2), step a) consists more specifically in printing a plurality of sensing elements (1) on at least one substrate (2).

This step is suitable for all configurations of the cementitious material structure of the present invention, in which the sensing elements (1) are formed *in situ*, and is not suitable for the cases in which these are prefabricated by means of any methods known in prior art, and just integrated *in situ* on the at least one substrate (2).

The printing step may consist of one of the following techniques, or combinations thereof:
- screen printing;
- rotogravure;
- ink jet printing in roller or sheets systems.

Encapsulation of the plurality of sensing elements (1) and the at least one substrate (2) may consist of a layer produced by lamination and/or coating with different materials - in order to obtain electrical, mechanical and chemical protection - after printing and before integration in the concrete structure and involvement with cementitious material. As protective materials, waterproof materials and materials with good mechanical and chemical resistance are considered in order to resist the concreting process of the concrete parts. The encapsulation of the printed sensors with these materials can be made through lamination, and/or heat sealing, and/or slot die, and/or doctor blade, and/or knife-over-edge, and/or screen printing, and/or spray, of a polymeric material curable by ultraviolet (UV) light and/or temperature.

In the case of the substrate (2) consists of a non-cementitious substrate, the method includes more specifically the following steps:
- phasing the concreting into outer and inner wall concreting;
- introduction of the plurality of sensing elements (1) printed on at least one substrate (2), after its encapsulation, into the formwork during the preparation of the inner wall concreting;

- in the introduction of previous step, positioning the plurality of sensing elements (1) printed on at least one substrate (2) with the aid of spacers made of cementitious material, and its attachment to the formwork reinforcement by means of spring systems or equivalent, in order to ensure stability and positioning during concreting and to comply with their actuation fields regarding the section of the part to be concreted;
- gradual introduction of the cementitious material so as not to damage the sensing systems and accessory parts.

Also in this case, the method more specifically includes the following steps:
- use of negatives in the parts, for placement and assembly of connecting boxes at strategic points previously defined in project;
- concreting the entire set with finishing concrete or coating concrete, depending on the dimensioning and the planned type of finishing.

The outer face is concreted according to the common procedure.

In the case, alternative to the above, of prefabrication of structures for end use - as is the case of street furniture - the method further includes the following steps of phased concreting by means of layers:
- in a first layer the concrete is placed and the set of the plurality of sensing elements (1) and the at least one substrate (2) is inserted under this layer;
- subsequently, another layer of concrete is placed.

These parts offer a finishing of the face under sight with architectural effects. Also in this case, negatives will be left in these parts to ensure the placement of accessories.

In a case which can be designated as mixed, wherein the plurality of sensing elements (1) is first incorporated into a prefabricated substrate (2) of cementitious material - such as micro concrete, mortar or compatible material - which encapsulates the sensors protecting them mechanically or against moisture and chemical attack, the method more specifically includes the following steps:
- pre-molding the cementitious material and printing the plurality of sensing elements (1), with all its connection points covered;
- placing the structure into the construction of the cementitious material structure with sensors - for instance a wall, floor, decorative part, or street furniture - prior to its concreting or final coating with the cementitious materials or others;
- attachment between the reinforcement and the formwork using support hooks and spacers in the case of elements to be concreted, or by means of gluing to the support in order to receive the final coating in the case of application under a plaster, screed or another coating.

Except for those configurations in which sensing elements (1) consist of BVP sensing elements or other type of sensor whose monitored variable is related to light, the sensing elements (1) are significantly close to the surface of the cementitious material structure with sensors, being covered with cementitious material and being at a depth of at least 0.5cm.

The present invention further relates to a method of operation of the cementitious material structure with sensors, which includes the following steps:
- detection of stimulus external to the cementitious material structure with sensors, through the plurality of sensing elements (1);
- conditioning the signals produced by the plurality of sensing elements (1);
- processing the signals from the plurality of sensing elements (1) in at least one microcontroller (8);
- actuation of the electronic element by the at least one microcontroller (8), wherein the electronic element is at least one actuator element (10) and/or a local and/or remote communication means (9).

### EMBODIMENTS

In an embodiment of the object of the present invention, the waterproofing material (3) is a polymeric material.

In another embodiment of the object of the present invention, the at least one substrate (2) consists of a polymeric substrate.

In one embodiment in which the sensing elements (1) are printed on the at least one substrate (2), each sensing element (1) includes a sensing element extension (11) for connection to the other electronic elements with functions such as signal conditioning or signal processing.

In one embodiment of the object of the present invention, the cementitious material consists of concrete, mortar or micro concrete.

In another embodiment of the present invention, the cementitious material consists of concrete with aesthetically appealing features, through using different colors/textures at least on the outer surface of the structure.

In different embodiments of the present invention, the printing ink includes conductive materials such as silver, carbon, nickel, gold, platinum, polymeric materials such as PEDOT:PSS.

In another embodiment which may be combined with the above, the cementitious material structure with sensors includes optical fibers, allowing light emission at the concrete surface without affecting their surface characteristics and durability. Such optical fibers are inserted during the concreting process, and allow the light emission at the concrete surface without affecting their surface characteristics and durability. These are the only elements placed at the surface of the cementitious material structure with sensors, of course besides the cementitious material itself.

In one embodiment of the present invention, the cementitious material structure with sensors consists of a blood volume pulse (BVP) meter, in which the sensing elements (1) are BVP sensors and the at least one actuator element (10) is a light emitting diode (LED).

In this case, the light emitters and receivers are in direct contact with the surface of the external element, as for example the finger of a user (the extremity of the body where the signal measurement is usually held in conventional systems), the conduction of the light being accomplished using optical fiber as previously described.

The integration of optical fibers is also carried out in order to generate dynamic effects by actuating the sensors through touch or proximity, at the concrete surface.

This integration is performed through the rigorous positioning of the fiber in the contact surface, in order to capture the light reflected by the skin after exposure to the light emitted by the emitting fiber. This positioning is pre-molded and subsequently embedded in the final element, or properly positioned in the formwork before concreting.

In different embodiments of the method of the present invention, the manufacture of the cementitious material structure with sensors may include artistic and architectural finishing, coloring and various textures which make its appearance decorative and pleasing, facilitating their integration in existing structures without damage and adding aesthetic value to them, and giving maximum comfort to users.

In a more specific embodiment relating the case in which the present invention includes a configuration of the cementitious material structure with sensors where the plurality of sensing elements (1) is arranged in such a way that a central sensing element (5) is surrounded by at least two peripheral sensors elements (6), the sensing elements (1) consist of different electrodes of a conductive material, such as silver electrodes printed on rigid or flexible substrates, in which they are arranged according to a given geometry.

These electrodes may be encapsulated to improve its strength and durability and to integrate them into the concrete, or materials can be used - preferably FR-4 - wherein the surface is coated with a conductive material (copper, silver, gold alloys, etc.). In these materials the electrodes are deployed in the geometrical arrangement necessary for the correct spatial signal detection (XYZ).

These electrodes are at least connected to the signal conditioning means (7), which in turn is connected to the at least one microcontroller (8).

In a preferred embodiment of the previous example, the at least one microcontroller (8) is connected to the local and/or remote communication means (9).

In the following, different examples of application of the structure and method of the present invention are presented.

### Example 1 - Development of switches and buzzers in concrete

In this example a method is considered with the following characteristics:
i) Integration of capacitive printed sensors in concrete, during the concreting process according to the method of the present invention.

The cementitious material structure with sensors specifically includes the following elements:
i) signal conditioning means (7), a microcontroller (8);
ii) at least one actuator element (10) consisting of a sound generating actuator and/or a lighting means actuator;
iii) the sensing elements (1) consist of touch or proximity capacitive sensors in the concrete surface;
iv) the at least one substrate (2) consists of flexible polymeric substrates where the capacitive sensors are printed and may be encapsulated to improve its strength and durability regarding its integration in concrete.

The ink used for printing includes conductive materials such as silver, carbon, nickel, gold or platinum, and polymeric materials such as PEDOT:PSS. As a printing technique for these capacitive sensors on the flexible polymeric substrates, rotogravure, or screen printing, or ink jet printing may be considered.

The substrate (2) consists of a polymeric film based in polyethylene terephthalate (PET). The protection of the sensor is obtained by using coating lamination, wet lamination and dry lamination.

The flexible polymeric substrates with printed sensors are integrated in the concrete during the concreting process.

This integration is performed in a process called bilayer, in which the sensor is integrated between layers, thus constituting a high mechanical strength pre-molded element with sensors, which may later be embedded in walls, floors or other constructive elements (integrated during the concreting or in posterior coatings with mortars or screeds) or simply be directly exposed in similar supports.

### Example 2 - Development of a concrete floor to security warnings

In this example a method is considered with the following characteristics:
i) Integration of printed piezoelectric sensors in concrete, during the concreting process according to the method of the present invention.

The cementitious material structure with sensors specifically includes the following elements:
i) signal conditioning means (7), a microcontroller (8);
ii) at least one actuator element (10) consisting of a sound generating actuator and/or a lighting means actuator;
iii) the sensing elements (1) consist of piezoelectric sensors in the concrete surface;
iv) optical fibers, in order the activation of the lighting means is visible at the concrete surface.

Piezoelectric sensors are identified for integration in the concrete. This type of sensors is encapsulated using epoxy resins, or others enabling the sensor protection against aggression of usage and concreting process.

The integration of the sensing elements (1) in concrete is carried out using the bilayer process of concreting, where the sensing elements (1) are integrated between the two layers.

The activation of the lighting means enables issuing a warning or message at the concrete surface. The control signal, for feedback at the respective concrete slab, can be obtained through the use of wiring (physical interconnection between the concrete slabs) or alternatively via wireless communication, for example via radio frequency (RF), Wi-Fi, Bluetooth, ZigBee, or other type of wireless communications protocol.

In a preferred embodiment, the ZigBee is used. This wireless communication protocol, considered as preferred, involves the use of a transceiver integrated on each of the concrete substrates (2), allowing them to communicate with each other.

As an application example, the floor issuing security alerts to warn drivers of a pedestrian approaching in crossing zones is considered.

In a preferred embodiment the lighting means are integrated in the concrete part, by using optical fibers which conduct the emitted light to the concrete surface, ensuring their characteristics and durability.

This set is remotely connected to a global management system for controlling the sensors remotely and customizing the type of interaction with the user and the way the actuator elements (10) act.

### Example 3 - Development of interactive games in concrete

In this example a method is considered with the following characteristics:
i) levelling the surface of the cementitious material - concrete;
ii) printing sensors at the concrete surface;
iii) integration of signal conditioning means (7), a microcontroller (8), and at least one actuator element (10) consisting of a sound generating actuator and/or a lighting means actuator;
iv) integration of optical fibers in the concrete, in order the activation of the lighting means is visible at the concrete surface.

This application includes the development of concrete parts with surface levelled by flattering and mechanical grinding after concreting, followed by sealing based on silanes-siloxanes and acrylic resins chemically compatible with the printing ink.

The printing of sensors at the concrete surface can be carried out using inks with conductive materials, for example silver, carbon, nickel, gold, platinum, polymeric materials such as PEDOT:PSS.

The printing is performed by rotogravure, or screen printing, or ink jet.

The sensor printed on the concrete surface is protected using waterproofing paints such as acrylic or silicate, or by means of micro concrete, mortar or compatible cementitious coating.

The technology or the integration of printed sensors on polymeric substrates, or the so called traditional sensing of concrete, allows developing interactive demonstrators for use as concrete parts in interactive street furniture, or flooring, or walls. These concrete parts can be applied to interactive games, through touch or proximity action.

### Example 4 - Reading the heartbeat by contact with the concrete surface

In this example a method is considered with the following characteristics:
i) Integration of printed BVP sensors in concrete, during the concreting process according to the method of the present invention.

The cementitious material structure with sensors specifically includes the following elements:
i) signal conditioning means (7), a microcontroller (8) and local and remote communication means (9);
ii) the sensing elements (1) consist of BVP sensors.

The integration of the BVP sensors in concrete is carried out by connecting the fibers ends embedded in the part.

This scheme has several possible applications, among which the most common is the heartbeat measurement through detection of peaks in the photoplethysmogram, but there may also exists applications in the detection of heart rate variability (HRV), or in the evaluation studies of arterial resistance and aorta elasticity.

Other applications of the sensor can be developed, since this allows obtaining vital information. For example, in studies of sleep disorders, the BVP sensors have been used to extract some parameters of arousal during sleep.

All these features, integrated in concrete, make it possible monitoring these parameters - for instance in private homes, nursing homes - by any user without the need for a specialized technician.

The data obtained are processed and monitored by a global management control system, and this data can be viewed remotely. If the values do not match the normal parameters (read values exceeding reference limits) an alert is sent to a user-defined contact.

As will be apparent to one skilled in the art, the present invention should not be limited to the embodiments described herein, and various changes are possible which remain within the scope of the present invention.

Of course, the preferred embodiments presented above can be combined in different possible ways, being herein avoided the repetition of all such combinations.

The invention should be limited only by the scope of the following claims.

## Claims

1. Cementitious material structure with sensors including at least one substrate (2), a plurality of sensing elements (1) arranged on the at least one substrate (2), and further including a waterproofing material layer (3) covering the set formed by the plurality of sensing elements (1) and the at least one substrate (2), **characterized in that** it further includes cementitious material involving the whole set formed by the said waterproofing material layer (3), at least one substrate and plurality of sensing elements (1), wherein the configuration, the arrangement and the sensor type allow said sensing elements to detect variables external to the cementitious structure based on interaction with a human user.

2. Structure according to claim 1, **characterized in that** the plurality of sensing elements (1) is arranged on a surface substantially parallel to an outer surface (4) of the cementitious material structure with sensors.

3. Structure according to any one of the preceding claims, **characterized in that** the plurality of sensing elements (1) is a plurality of sensing elements (1) printed on the at least one substrate (2) and **in that** the at least one substrate preferably consists of:
• a non-cementitious material substrate, which preferably consists of a polymeric material, glass, paper, derivatives of paper, or ceramic material, or
• the cementitious material itself or a micro concrete layer, and a printing surface of the plurality of sensing elements (1) forms in cementitious material a substantially finite plane parallel to the said outer surface (4) of the structure.

4. Structure according to the preceding claim **characterized in that** the sensing elements (1) consist of capacitive and inductive sensors, sensors of electro-dermal activity, temperature, luminosity, or sound.

5. Structure according to the previous claim wherein the sensing elements (1) consist of blood volume pulse sensors and by including optical fibers arranged in such a way that one of the ends of each optical fiber is at the outer surface (4) of the structure and the other end is at the surface of each sensing element (1).

6. Structure according to any one of the preceding claims, **characterized in that** the cementitious material is concrete, mortar or shotcrete.

7. Structure according to any one of the preceding claims, **characterized in that** it includes several planes parallel to each other and to the outer surface, which in turn includes a plurality of sensing elements (1), defining a three-dimensional mesh of sensing elements (1), wherein the plurality of sensing elements (1) is preferably arranged in such a way that a central sensing element (5) is surrounded by at least two peripheral sensing elements (6), with all the sensing elements (1) arranged on a line which is part of a plane parallel to the surface of the cementitious material structure with sensors.

8. Structure according to the preceding claim, **characterized in that** it includes at least four peripheral sensing elements (6) arranged in pairs in at least two perpendicular lines which are part of a plane parallel to the surface of the cementitious material structure with sensors and intersect at midpoint of the central sensing element (5) and, optionally, the waterproofing material (3) being a polymer.

9. Structure according to any one of the preceding claims, **characterized in that** it includes signal conditioning means (7), at least one microcontroller (8), and, optionally:
• local or remote communication means (9), and/or
• at least one actuator element (10) and optical fibers arranged in such a way that one of the ends of each optical fiber is at the outer surface (4) of the structure and the other end is at the surface of each actuator element (10), the actuator elements (10) consisting of light generating elements.

10. Method for manufacturing a cementitious material structure with sensors of any one of the preceding claims wherein it includes the following steps:
a) integrating a plurality of sensing elements (1) on the at least one substrate (2) and, subsequently, covering by encapsulation the set formed by the at least one substrate and plurality of sensing elements (1) with a waterproofing material layer (3) and involving the whole set formed by the said waterproofing material layer (3), the plurality of sensing elements (1) and the at least one substrate (2) with a cementitious material;
b) inserting the said whole set involved in the cementitious material inside a formwork;
c) filling the formwork resulting from step b) with cementitious material, wherein the said sensing elements are so arranged that they can detect variables external to the cementitious structure based on interaction with a human user.

11. Method according to the previous claim, **characterized in that**
step a) consists more specifically of printing a plurality of sensing elements (1) on at least one substrate (2) wherein, preferably, printing consists of one of the following techniques, or combinations thereof:
- screen printing;
- rotogravure;
- ink jet printing in roller or sheets systems.

12. Manufacturing method according to any of the claims 10 and 11, **characterized in that,** in case the substrate (2) consists of a non-cementitious substrate, it includes the following steps:
- phasing the concreting into outer and inner wall concreting;
- introduction of the plurality of sensing elements (1) printed on at least one substrate (2), after its encapsulation, into the formwork during the preparation of the inner wall concreting;
- in the introduction of previous step, positioning the plurality of sensing elements (1) printed on at least one substrate (2) with the aid of spacers made of cementitious material, and its attachment to the formwork reinforcement by means of spring systems or equivalent;
- gradual introduction of the cementitious material so as not to damage the sensing elements and accessory parts;
- use of negatives in the parts, for placement and assembly of connecting boxes at strategic points previously defined in project;
- concreting the entire set with finishing concrete or coating concrete, depending on the dimensioning and the planned type of finishing.

13. Manufacturing method according to any of the claims 10 and 11, **characterized in that,** in case the substrate (2) consists in the cementitious material itself, the concreting is phased by layers of prefabricated structures for final use, including the following steps:
- placing the concrete in a first layer and inserting under this layer the set of the plurality of sensing elements (1) and the at least one substrate (2);
- placing thereafter another concrete layer.

14. Manufacturing method according to any of the claims 10 and 11, **characterized in that** the plurality of sensing elements (1) is previously incorporated in a prefabricated substrate (2) of cementitious material - such as micro concrete, mortar or compatible material - further including the following steps:
- pre-molding the cementitious material and printing the plurality of sensing elements (1), with all its connection points covered;
- placing the structure into the construction of the cementitious material structure with sensors for instance a wall, floor, decorative part, or street furniture - prior to its concreting or final coating with the cementitious materials or others;
- attachment between the reinforcement and the formwork using support hooks and spacers in the case of element to be concreted, or by means of gluing to the support in order to receive the final coating in the case of application under a plaster, screed or another coating.

15. Method for operating the cementitious material structure with sensors of claims 1 to 9, wherein it includes the following steps:
- detection of a stimulus external to the cementitious material structure with sensors, through the plurality of sensing elements (1) and thereby producing signals;
- conditioning the said signals produced by the plurality of sensing elements (1);
- processing the signals from the plurality of sensing elements (1) in at least one microcontroller (8);
- actuating an electronic element by the at least one microcontroller (8), wherein the electronic element is at least one actuator element (10) and/or a local or remote communication means (9).

## Patentansprüche

1. Struktur aus zementhaltiges Material mit Sensoren, die mindestens ein Substrat (2), eine Vielzahl von Sensorelementen (1), die auf dem mindestens einen Substrat (2) angeordnet sind, und außerdem eine Abdichtungsmaterial Schicht (3) umfasst, die die durch die Vielzahl von Sensorelementen (1) und das mindestens eine Substrat (2) gebildete Einheit bedeckt, **dadurch gekennzeichnet, dass** sie ferner zementhaltige Material umfasst, das die gesamte Einheit einschließt, die durch die Abdichtungsmaterial Schicht (3), mindestens ein Substrat und eine Vielzahl von Sensorelementen (1) gebildet wird, wobei die Konfiguration, die Anordnung und der Sensortyp es den Sensorelementen ermöglichen, Variablen außerhalb der zementhaltige Struktur auf der Grundlage der Interaktion mit einem menschlichen Benutzer zu erfassen.

2. Struktur nach Anspruch 1, **dadurch gekennzeichnet, dass** die Vielzahl von Sensorelementen (1) auf einer Fläche angeordnet ist, die im Wesentlichen parallel zu einer Außenfläche (4) der Struktur aus zementhaltiges Material mit Sensoren verläuft.

3. Struktur nach einem der vorhergehenden Ansprüche, **dadurch gekennzeichnet, dass** die Vielzahl von Sensorelementen (1) eine Vielzahl von Sensorelementen (1) ist, die auf das mindestens eine Substrat (2) gedruckt sind, und dass das mindestens eine Substrat vorzugsweise besteht aus:
• ein Substrat aus nicht-zementhaltiges Material, das vorzugsweise aus einem polymeren Material, Glas, Papier, Papierderivaten oder keramischem Material besteht, oder
• das zementhaltige Material selbst oder eine Mikrobetonschicht, und eine Druckfläche der Vielzahl von Sensorelementen (1) bildet im zementhaltigen Material eine im Wesentlichen endliche Ebene parallel zur genannten Außenfläche (4) der Struktur.

4. Struktur nach dem vorhergehenden Anspruch, **dadurch gekennzeichnet, dass** die Sensorelemente (1) aus kapazitiven und induktiven Sensoren, Sensoren für elektrodermale Aktivität, Temperatur, Helligkeit oder Schall bestehen.

5. Struktur nach dem vorhergehenden Anspruch, wobei die Sensorelemente (1) aus Blutvolumen-Puls-Sensoren bestehen und optische Fasern enthalten, die so angeordnet sind, dass eines der Enden jeder optischen Faser an der Außenfläche (4) der Struktur und das andere Ende an der Oberfläche jedes Sensorelements (1) liegt.

6. Struktur nach einem der vorhergehenden Ansprüche, **dadurch gekennzeichnet, dass** das zementhaltige Material Beton, Mörtel oder Spritzbeton ist.

7. Struktur nach einem der vorhergehenden Ansprüche, **dadurch gekennzeichnet, dass** sie mehrere zueinander und zur äußeren Oberfläche parallele Ebenen umfasst, die wiederum eine Vielzahl von Sensorelemente (1) umfassen, die ein dreidimensionales Netz von Sensorelemente (1) definieren, wobei die Vielzahl von Sensorelemente (1) vorzugsweise so angeordnet ist, dass ein zentrales Sensorelement (5) von mindestens zwei peripheren Sensorelemente (6) umgeben ist, wobei alle Sensorelemente (1) auf einer Linie angeordnet sind, die Teil einer zur Oberfläche der Struktur aus zementhaltiges Material mit Sensoren parallelen Ebene ist.

8. Struktur nach dem vorhergehenden Anspruch, **dadurch gekennzeichnet, dass** sie mindestens vier periphere Sensorelemente (6) umfasst, die paarweise in mindestens zwei senkrechten Linien angeordnet sind, die Teil einer zur Oberfläche der Struktur aus zementhaltiges Material mit Sensoren parallelen Ebene sind und sich in der Mitte des zentralen Sensorelements (5) schneiden, und wobei das Abdichtungsmaterial (3) gegebenenfalls ein Polymer ist.

9. Struktur nach einem der vorhergehenden Ansprüche, **dadurch gekennzeichnet, dass** sie Signalaufbereitungsmittel (7), mindestens einen Mikrocontroller (8) und optional Folgendes umfasst:
• lokale oder entfernte Kommunikationsmittel (9), und/oder
• mindestens ein Betätigungselement (10) und optische Fasern, die so angeordnet sind, dass eines der Enden jeder optischen Faser an der Außenfläche (4) der Struktur und das andere Ende an der Oberfläche jedes Betätigungselements (10) liegt, wobei die Betätigungselemente (10) aus lichterzeugenden Elementen bestehen.

10. Verfahren zur Herstellung einer Struktur aus zementhaltiges Material mit Sensoren von nach einem der vorhergehenden Ansprüche, **wobei** es die folgenden Schritte umfasst:
a) Integrieren einer Vielzahl von Sensorelementen (1) auf dem mindestens einen Substrat (2) und anschließendes Abdecken des aus dem mindestens einen Substrat und der Vielzahl von Sensorelementen (1) gebildeten Satzes durch Einkapseln mit einer Abdichtungsmaterial Schicht (3) und Einschließen des gesamten Einheit, der durch die Abdichtungsmaterial Schicht (3), der Vielzahl von Sensorelementen (1) und dem mindestens einen Substrat (2) gebildet wird, mit einem zementhaltigen Material;
b) Einlegen des gesamten Einheit in das zementhaltige Material einschließen innerhalb einer Schalung;
c) Füllen der aus Schritt b) resultierenden Schalung mit zementhaltigem Material, wobei die genannten Sensorelemente so angeordnet sind, dass sie auf der Grundlage der Interaktion mit einem menschlichen Benutzer Variablen außerhalb der zementhaltigen Struktur erfassen können.

11. Verfahren nach dem vorhergehenden Anspruch, **dadurch gekennzeichnet, dass**
Schritt a) besteht insbesondere darin, eine Vielzahl von Sensorelementen (1) auf mindestens ein Substrat (2) zu drucken, wobei das Drucken vorzugsweise aus einer der folgenden Techniken oder Kombinationen davon besteht:
- Siebdruck;
- Tiefdruck;
- Tintenstrahldruck in Walzen- oder Bogensystemen.

12. Herstellungsverfahren nach einem der Ansprüche 10 und 11, **dadurch gekennzeichnet, dass** es für den Fall, dass das Substrat (2) aus einem nicht-zementhaltiges Substrat besteht, die folgenden Schritte umfasst:
- Aufteilung der Betonierung in Außen- und Innenwandbetonierung;
- Einführung der Vielzahl der auf mindestens ein Substrat (2) gedruckten Sensorelemente (1) nach deren Einkapseln in die Schalung während der Vorbereitung der Innenwandbetonierung;
- in der Einführung des vorhergehenden Schritts, Positionierung der Vielzahl von Sensorelementen (1), die auf mindestens ein Substrat (2) gedruckt sind, mit Hilfe von Abstandshaltern aus zementhaltiges Material und ihre Befestigung an der Schalungsbewehrung mittels Federsystemen oder Ähnlichem;
- allmähliches Einführung des zementhaltigen Materials, um die Sensorelemente und Zubehörteile nicht zu beschädigen;
- Verwendung von Negativen in den Teilen, für die Platzierung und Montage von Verbindungsboxen an strategischen Punkten, die zuvor im Projekt festgelegt wurden;
- Betonieren des gesamten Satzes mit Fertigbeton oder Beschichtungsbeton, je nach Dimensionierung und geplanter Art der Endbearbeitung.

13. Herstellungsverfahren nach einem der Ansprüche 10 und 11, **dadurch gekennzeichnet, dass** in dem Fall, dass das Substrat (2) aus das zementhaltige Material selbst besteht, das Betonieren durch Schichten von vorgefertigten Strukturen für die endgültige Verwendung phasenweise erfolgt, was die folgenden Schritte umfasst:
- Einbringen des Betons in einer ersten Schicht und Einlegen des Satzes aus der Vielzahl von Sensorelementen (1) und dem mindestens einem Substrat (2) unter diese Schicht;
- Danach wird eine weitere Betonschicht eingebracht.

14. Herstellungsverfahren nach einem der Ansprüche 10 und 11, **dadurch gekennzeichnet, dass** die Vielzahl von Sensorelementen (1) zuvor in ein vorgefertigtes Substrat (2) aus zementhaltiges Material - wie Mikrobeton, Mörtel oder kompatiblen Material - eingearbeitet wird, das außerdem die folgenden Schritte umfasst:
- Vorformen des zementhaltigen Materials und Bedrucken der Vielzahl von Sensorelementen (1), wobei alle ihre Verbindungspunkte abgedeckt werden;
- Einbringen der Struktur in die Konstruktion der Struktur aus zementhaltiges Material mit Sensoren, z. B. einer Wand, eines Bodens, eines dekorativen Teils oder einer Stadtmöblierung - vor dem Betonieren oder der endgültigen Beschichtung mit den zementhaltigen Materialien oder anderen;
- Befestigung zwischen der Bewehrung und der Schalung mit Hilfe von Stützhaken und Abstandshaltern im Falle eines zu betonierenden Elements oder durch Verkleben auf der Unterlage zur Aufnahme der endgültigen Beschichtung im Falle der Anwendung unter einem Putz, Estrich oder einer anderen Beschichtung.

15. Verfahren zum Betreiben der Struktur aus zementhaltiges Material mit Sensoren nach einem der Ansprüche 1 bis 9, **wobei** es die folgenden Schritte umfasst:
- Erfassen eines Reizes außerhalb der Struktur aus zementhaltiges Material mit Sensoren durch die Vielzahl von Sensorelementen (1) und dadurch Erzeugen von Signalen;
- Aufbereitung der von der Vielzahl der Sensorelemente (1) erzeugten Signale;
- Verarbeitung der Signale von der Vielzahl von Sensorelementen (1) in mindestens einem Mikrocontroller (8) ;
- Ansteuerung eines elektronischen Elements durch den mindestens einen Mikrocontroller (8), wobei das elektronische Element mindestens ein Betätigungselement (10) und/oder ein lokales oder entferntes Kommunikationsmittel (9) ist.

## Revendications

1. Structure de matériau cimentaire avec des capteurs comprenant au moins un substrat (2), une pluralité d'éléments de détection (1) disposés sur le au moins un substrat (2), et comprenant en outre une couche de matériau imperméabilisant (3) recouvrant l'ensemble formé par la pluralité d'éléments de détection (1) et le au moins un substrat (2), **caractérisée en ce qu'**elle comprend en outre un matériau cimentaire enveloppant tout l'ensemble formé par ladite couche de matériau imperméabilisant (3), le au moins un substrat et la pluralité d'éléments de détection (1), dans laquelle la configuration, la disposition et le type de capteur permettent auxdits éléments de détecter des variables externes à la structure cimentaire sur la base d'une interaction avec un utilisateur humain.

2. Structure selon la revendication 1, **caractérisée en ce que** la pluralité d'éléments de détection (1) est disposée sur une surface substantiellement parallèle à une surface extérieure (4) de la structure de matériau cimentaire avec des capteurs.

3. Structure selon l'une quelconque des revendications précédentes, **caractérisée en ce que** pluralité d'éléments de détection (1) est une pluralité d'éléments de détection (1) imprimés sur le au moins un substrat (2) et **en ce que** le au moins un substrat est de préférence constitué :
• d'un substrat de matériau non cimentaire, qui consiste de préférence en un matériau polymère, du verre, du papier, des dérivés du papier ou un matériau céramique, ou
• du matériau cimentaire lui-même ou d'une couche de microbéton, et une surface d'impression de la pluralité d'éléments de détection (1) forme dans le matériau cimentaire un plan substantiellement fini parallèle à ladite surface extérieure (4) de la structure.

4. Structure selon la revendication précédente, **caractérisée en ce que** les éléments de détection (1) consistent en des capteurs capacitifs et inductifs, d'activité électrodermale, de température, de luminosité ou sonores.

5. Structure selon la revendication précédente, dans laquelle les éléments de détection (1) consistent en des capteurs de pression sanguine volumique et qui inclut des fibres optiques disposées de telle sorte que l'une des extrémités de chaque fibre optique se trouve à la surface extérieure (4) de la structure et l'autre extrémité se trouve à la surface de chaque élément de détection (1).

6. Structure selon l'une quelconque des revendications précédentes, **caractérisée en ce que** le matériau cimentaire est le béton, le mortier ou le béton projeté.

7. Structure selon l'une quelconque des revendications précédentes, **caractérisée en ce qu'**elle comprend plusieurs plans parallèles entre eux et à la surface extérieure, qui comprend à son tour une pluralité d'éléments de détection (1), définissant un maillage tridimensionnel d'éléments de détection (1), où la pluralité d'éléments de détection (1) est de préférence disposée de telle sorte qu'un élément de détection central (5) est entouré d'au moins deux éléments de détection périphériques (6), tous les éléments de détection (1) étant disposés sur une ligne qui fait partie d'un plan parallèle à la surface de la structure de matériau cimentaire avec des capteurs.

8. Structure selon la revendication précédente, **caractérisée en ce qu'**elle comprend au moins quatre éléments de détection périphériques (6) disposés par paires sur au moins deux lignes perpendiculaires qui font partie d'un plan parallèle à la surface de la structure de matériau cimentaire avec des capteurs et se croisent au milieu de l'élément de détection central (5) et, facultativement, le matériau d'imperméabilisation (3) étant un polymère.

9. Structure selon l'une quelconque des revendications précédentes, **caractérisée en ce qu'**elle comprend des moyens de conditionnement de signaux (7), au moins un microcontrôleur (8) et, facultativement :
• des moyens de communication locale ou à distance (9), et/ou
• au moins un élément actionneur (10) et des fibres optiques disposées de telle sorte que l'une des extrémités de chaque fibre optique se trouve à la surface extérieure (4) de la structure et l'autre extrémité se trouve à la surface de chaque élément actionneur (10), les éléments actionneurs (10) consistant en des éléments de génération de lumière.

10. Procédé de fabrication d'une structure de matériau cimentaire avec des capteurs de l'une quelconque des revendications précédentes, lequel comprend les étapes suivantes :
a) intégrer une pluralité d'éléments de détection (1) sur le au moins un substrat (2) et, ensuite, recouvrir par encapsulation l'ensemble formé par le au moins un substrat et la pluralité d'éléments de détection (1) avec une couche de matériau imperméabilisant (3) et envelopper tout l'ensemble formé par ladite couche de matériau imperméabilisant (3), la pluralité d'éléments de détection (1) et le au moins un substrat (2) avec un matériau cimentaire ;
b) insérer ledit ensemble enveloppé par le matériau cimentaire à l'intérieur d'un coffrage ;
c) remplir le coffrage résultant de l'étape b) avec un matériau cimentaire, où lesdits éléments de détection sont disposés de manière à pouvoir détecter des variables externes à la structure cimentaire sur la base d'une interaction avec un utilisateur humain.

11. Procédé selon la revendication précédente, **caractérisé en ce que** l'étape a) consiste plus précisément à imprimer une pluralité d'éléments de détection (1) sur au moins un substrat (2), l'impression se faisant de préférence selon l'une des techniques suivantes, ou des combinaisons de celles-ci :
- sérigraphie ;
- rotogravure ;
- impression à jet d'encre dans des systèmes à rouleaux ou à feuilles ;

12. Procédé de fabrication selon l'une quelconque des revendications 10 et 11, **caractérisé en ce que**, dans le cas où le substrat (2) consiste en un substrat non cimentaire, il comprend les étapes suivantes :
- le phasage du bétonnage en bétonnage de la paroi extérieure et de la paroi intérieure ;
- l'introduction à l'intérieur du coffrage de la pluralité d'éléments de détection (1) imprimés sur au moins un substrat (2), après leur encapsulation, pendant la préparation du bétonnage de la paroi intérieure ;
- lors de l'introduction de l'étape précédente, le positionnement de la pluralité d'éléments de détection (1) imprimés sur au moins un substrat (2) à l'aide d'entretoises en matériau cimentaire, et leur fixation à l'armature du coffrage au moyen de systèmes de ressorts ou équivalents ;
- l'introduction progressive du matériau cimentaire de manière à ne pas endommager les éléments de détection et les parties accessoires ;
- l'utilisation de négatifs dans les pièces, pour la mise en place et l'assemblage de boîtes de connexion aux points stratégiques préalablement définis dans le projet ;
- le bétonnage de l'ensemble avec du béton de finition ou du béton de revêtement, en fonction du dimensionnement et du type de finition prévu.

13. Procédé de fabrication selon l'une quelconque des revendications 10 et 11, **caractérisé en ce que**, dans le cas où le substrat (2) consiste en le matériau cimentaire lui-même, le bétonnage est échelonné par des couches de structures préfabriquées pour l'utilisation finale, comprenant les étapes suivantes :
- placer le béton dans une première couche et insérer sous cette couche l'ensemble de la pluralité d'éléments de détection (1) et le au moins un substrat (2) ;
- placer ensuite une autre couche de béton.

14. Procédé de fabrication selon l'une quelconque des revendications 10 et 11, **caractérisé en ce que** la pluralité d'éléments de détection (1) est préalablement incorporée dans un substrat préfabriqué (2) de matériau cimentaire - tel que le microbéton, le mortier ou un matériau compatible - comprenant en outre les étapes suivantes :
- le pré-moulage du matériau cimentaire et l'impression de la pluralité d'éléments de détection (1), tous les points de connexion étant couverts ;
- le placement de la structure dans la construction de la structure de matériau cimentaire avec des capteurs, par exemple un mur, un sol, une pièce décorative ou un mobilier urbain, avant son bétonnage ou son revêtement final avec les matériaux cimentaires ou autres ;
- la fixation entre l'armature et le coffrage à l'aide de crochets de support et d'entretoises dans le cas d'un élément à bétonner, ou par collage au support pour recevoir le revêtement final dans le cas d'une application sous un enduit, une chape ou un autre revêtement.

15. Procédé d'opération de la structure de matériau cimentaire avec des capteurs des revendications 1 à 9, lequel comprend les étapes suivantes :
- la détection d'un stimulus externe à la structure de matériau cimentaire avec des capteurs, à travers la pluralité d'éléments de détection (1) et produisant ainsi des signaux ;
- le conditionnement desdits signaux produits par la pluralité d'éléments de détection (1) ;
- le traitement des signaux provenant de la pluralité d'éléments de détection (1) dans au moins un microcontrôleur (8) ;
- l'actionnement d'un élément électronique par l'intermédiaire du au moins un microcontrôleur (8), l'élément électronique étant au moins un élément actionneur (10) et/ou un moyen de communication locale ou à distance (9) .
